# EUROPEAN PATENT APPLICATION

(11) **EP 2 664 607 A1**
(43) Date of publication of application: **20.11.2013**
(21) Application number: 12168332.0
(22) Date of filing: 16.05.2012
(51) Int. Cl.: C07C 51/09, C07C 51/363, C07C 53/21, C07C 57/54, C07C 67/307, C07C 69/63, C07C 69/653

(54) **Fluorination process**

(71) Applicant: SOLVAY SA, 1120 Bruxelles (BE)
(72) Inventor: Braun, Max Josef, 30900 WEDEMARK (DE)
(74) Representative: Mross, Stefan P.M.

(57) **Abstract**

Process for the manufacture of a fluoroorganic compound, which comprises reacting fluorine gas with an organic compound comprising at least one non-(hetero)aromatic unsaturated bond, in a microreactor in the presence of a solvent.

## Description

The present invention concerns a process for the manufacture of fluorinated compounds.

Fluorinated compounds have widespread use, for example as refrigerants, as blowing agents for polymer foams, as building blocks for agrochemicals, as monomers for fluoropolymers and a lot of further applications. Certain fluorinated olefins are useful as monomers for medical polymers.

EP-A-383128 describes the fluorination of certain acrylic acid derivatives with elemental fluorine in a solvent, notably CFC-12 (difluorodichloromethane). 2,3-difluoropropionic acid derivatives obtained are converted into corresponding 2-fluoroacrylic acid derivatives.

The invention concerns a process for the manufacture of a fluoroorganic compound, which comprises reacting elemental fluorine (F2) with an organic compound comprising at least one non-(hetero)aromatic unsaturated bond, in a microreactor in the presence of a solvent.

Surprisingly, by the process according to the invention, fluorination reactions of organic compound comprising at least one non-(hetero)aromatic unsaturated bond can be carried our selectively and efficiently. Side reactions, in particular polymerization of the generally polymerization sensitive organic compound can be avoided, at least to the extent necessary to avoid plugging of the microreactor. Higher reaction temperatures can be used, allowing for reduced energy consumption. Prior art CFC solvents, having ozone depletion potential, can be avoided.

Microreactors are known in the art (cf. e.g. WO 2007/042313 A2, US 2009/0295005 A1, EP 1 481 724 A2, or WO 2007/027785 A2). "Microreactors" as used herein is understood in the broadest technical meaningful sense. In the art often "micromixer" and "microreactor" are used as synonyms. In some cases, however, a microreactor which mixes a plurality of fluids together is called a "micromixer" and a microreactor which causes a chemical reaction during the mixing of a plurality of fluids is called a "microreactor". The microreactor as used herein is a device comprising "micromixers", "microreactors" and combinations of these as used in the art. Preferably, "microreactor" as used herein is a device which comprises components, typically channels or flow ducts, having characteristic/determining geometric dimensions of 1 µm to 2000 µm, and in particular preferable from 10 µm to 1000 µm A microreactor is typically provided with a reaction channel which leads to a plurality of fine reaction channels or flow ducts. The equivalent diameter obtained in the section of the fine reaction channel is, converted to a circle, several micrometers to several hundreds of micrometers.

Preferably, "microreactor" as used herein includes at least one micromixer, which is preferably used in combination with a further microreactor. Further, the term "microreactor" used herein can be a device, which is referred to in the art as "minireactor", "micro heat exchanger", "minimixer" or "micromixer". Examples of these are microreactors, micro heat exchangers, and T- and Y-mixers, as available by a large number of companies (e.g. Ehrfeld Mikrotechnik BTS GmbH, Institut für Mikrotechnik Mainz GmbH, Siemens AG, CPC-Cellulare Process Chemistry Systems GmbH).

In a preferred embodiment, "microreactor" comprises a known static micromixer, more preferably a multi-lamination mixer, split and recombine mixers, or else mixers with a cross-sectional constriction. Preferably, the static micromixers are flowed through continuously. Examples of known static micromixers are stack mixers as described in DE 202 06 371 U1 slotted plate mixers, as described in WO 2004/052518 A2, or else comb mixers, as described e.g. in DE 202 09 009 U1, as available by, for example, Ehrfeld Mikrotechnik BTS GmbH. These are multi-lamination mixers, in which the two fluid streams to be mixed are fanned out into a multitude of thin lamellae or films, and these lamellae are then merged with one another in alternation, such that the diffusion and secondary flows result in rapid mixing.

As alternative micromixers, V-type mixers, as available by Forschungszentrum Karlsruhe, split and recombine mixers, e.g. cascade mixers or faceted mixers, as available by Ehrfeld Mikrotechnik BTS GmbH, or caterpillar mixers, e.g. obtainable from the Institut für Mikrotechnik, Mainz, can be used. In these mixers the product streams to be mixed are divided into smaller flows and these smaller flows are repeatedly combined and divided. Further alternative micromixers with a cross-sectional constriction, such as focus mixers or cyclone mixers, or else jet mixers, as described in EP 1 165 224 B1, e.g. obtainable from Synthesechemie, and impingement jet mixers or valve mixers, as described in WO 2005/079964 A1, available from Ehrfeld Mikrotechnik BTS GmbH, can be used. Particular preferred cascade mixers are used as micromixers in the process of the present invention.

In a preferred embodiment the microreactors comprise channels with channel widths of less than 1000 µm, preferably less than 500 µm, more preferably less than 200 µm, in particular less than 100 µm Preferably the microreactor applied in the process according to the present invention comprises a micromixer, and more preferably comprises a combination of a micromixer with a further microreactor, most preferably a sandwich type microreactor. In one preferred embodiment, the further microreactor used is a micro heat exchanger or a microreactor having an integrated static mixing function, in which the reaction can be carried out under defined flow conditions, such as low axial and good radial mixing. This results in a narrow residence time distribution, in particular in continuous operation. Preferably a sandwich type reactor from Ehrfeld Mikrotechnik BTS GmbH is used, more preferably in combination with a micromixer, in particular a cascade mixer. The temperature of the microreactors as used herein can preferably be controlled by use of a temperature control fluid circulating through and/or around the microreactor. In a preferred embodiment "microreactor" comprises a static mixer, preferably a microstructured static mixer, in particular the mixers as specified above, most preferably in combination with a further microreactor, in particular a sandwich type microreactor.

In a preferred embodiment known microstructured heat exchangers are used as microreactors. These known heat exchangers include micro-heat exchangers which consist of stacked thin plates provided with microchannels and welded to one another. Microstructure heat exchangers are, e.g. cross-current or counter-current plate heat exchangers as described in DE 37 09 278 A1. Such heat exchangers are available from Ehrfeld Mikrotechnik BTS GmbH. Preferably the microreactor applied in the process according to the present invention is a combination of a micromixer as described above and a microstructured heat exchanger.

In a preferred embodiment of the process according to the present invention, the microreactor, in particular the static micromixer is flowed through continuously. Thereby, the reaction mixture can advantageously be kept at the optimal reaction temperature of around 0°C to 40°C, preferably from 10°C to 30°C, more preferably about 25°C or about 20°C. The high heat transfer performance of the microreactors, in particular of the microstructured heat exchangers, thereby guarantees that the temperature can be kept in the optimal range, in particular also during the first stages of the reaction, where typically the most reaction heat (enthalpy) is generated. In the micromixers the small volumes of the reaction mixture allow exact temperature control and thereby prevent an undesired rise in the temperature which typically leads to side reactions and loss in selectivity. In addition, the efficiency of the process is enhanced, since, owing to the very high exchange area per unit volume, it is possible to work with more highly concentrated reaction and work-up mixtures. The intensive mixing of the phases additionally leads to acceleration of the preparation and optional work-up reaction, and to improved reaction conversion and increase in yield and selectivity of the reaction.

In a more preferred embodiment, the microreactor comprises at least one microstructure heat exchanger.

In the process according to the invention, the organic compound usually comprises at least one olefinic double bond. Often, the organic compound comprises in addition at least one functional group. Examples of functional groups include a -C=O function, a -C=N-R' function wherein R' is H, alkyl or aryl, and a -C=S function. These latter functions are preferably in conjugation with the at least one unsaturated bond.

In a particular aspect, the organic compound corresponds to formula

HR1C=CR2-(C=O)-R3 (I)

wherein R1 and R2 are independently selected from H, optionally fluorinated, alkyl, (hetero)aryl, aralkyl and halogen, preferably fluorine or chlorine, and R3 is selected from, optionally fluorinated, alkyl, (hetero)aryl, aralkyl, OR4, wherein R4 is H, optionally fluorinated, alkyl, (hetero)aryl or aralkyl and NR5R6, wherein R5 and R6 are independently selected from H, optionally fluorinated, alkyl, (hetero)aryl and aralkyl.

In the present invention, "alkyl" is understood to denote in particular a straight chain, branched chain, or cyclic hydrocarbon groups usually having from 1 to 20 carbon atoms. Examples of alkyl groups include methyl, ethyl, propyl, isopropyl, butyl, isobutyl, sec-butyl, tert-butyl, pentyl, neopentyl, hexyl, cyclopropyl, cyclobutyl, cyclopentyl, and cyclohexyl.

In the present invention, "aryl" is understood to denote in particular a substituent comprising an aromatic ring. Generally, the aryl groups have 6 to 20 carbon atoms, preferably 6 to 12 carbon atoms. The aryl groups are preferably optionally substituted phenyl groups, naphthyl groups, anthryl group and phenanthryl group.

"Heteroaryl" is understood to denote in particular a substituent comprising an aromatic ring in which at least one ring atom is a heteroatom, preferably selected from nitrogen, oxygen and sulphur.

"Aralkyl" is understood to denote in particular a methylene group substituted by an aryl group or a heteroaryl group as described here before, in particular a benzyl group.

Any of R1, R2, R3, R4, R5 or R6, especially R1, R4, R5 or R6 can be selected from fluorinated alkyl, fluorinated (hetero)aryl and fluorinated aralkyl groups. A fluorinated alkyl group is preferred. Often, A fluorinated C₁-C₄ alkyl group is more particularly preferred. It is especially preferred if the fluorinated alkyl is methyl, ethyl or methyl or ethyl substituted by at least one fluorine atom. CF₃, CF₂H, CF₂C1, C₂F₅, C₃F₇ are particularly preferred especially as R4, R5 and R6. CF₃, CF₂C1 and CF₂H are most particularly preferred.

In a preferred embodiment, the organic compound corresponds to formula

H2C=CH-(C=O)-OR4 (II)

wherein R4 is selected from H and, optionally fluorinated, methyl, ethyl, n-propyl, isopropyl, n-butyl, isobutyl and tert.butyl.

In a first particular aspect of the preferred embodiment, R4 is methyl or ethyl, preferably methyl.

In a second particular aspect of the preferred embodiment, R4 is a fluorinated C1-C4 alkyl as described here above.

In the process according to the invention, the fluororganic compound obtained through the reaction with elemental fluorine is generally an α,β-difluoro-addition product to the unsaturated bond. When the reaction is carried out on an organic compound of formula (I), then the α,β-difluoro-addition product corresponds to formula

HR1FC-CFR2-(C=O)-R3 (III)

wherein R1 and R2 are independently selected from H, optionally fluorinated, alkyl, (hetero)aryl, aralkyl and halogen, preferably fluorine or chlorine, and R3 is selected from, optionally fluorinated, alkyl, (hetero)aryl, aralkyl, OR4, wherein R4 is H, optionally fluorinated, alkyl, (hetero)aryl or aralkyl and NR5R6, wherein R5 and R6 are independently selected from H, optionally fluorinated, alkyl, (hetero)aryl and aralkyl. All definitions and preferences for R1, R2, R3, R4, R5 or R6 are as described above.

When the reaction is carried out on an organic compound of formula (II), then the α,β-diftuoro-addition product corresponds to formula

H2C=CH-(C=O)-OR4 (IV)

wherein R4 is selected from H and, optionally fluorinated, methyl, ethyl, n-propyl, isopropyl, n-butyl, isobutyl and tert.butyl, All definitions and preferences for R4 are as described above.

In the process according to the invention, the solvent is generally substantially inert to reaction with elemental fluorine under the conditions of the reaction. "Substantially inert" is understood to denote the fact that after the reaction, not more than 10 % molar, preferably not more than 5 % more preferably not more than 1 % of the solvent has reacted with elemental fluorine. Preferred solvents are fluorinated compounds having no ozone depletion potential. Particularly, the solvent is selected from the group consisting of perfluorocarbons, in particular perfluorohexane, perfluoro(poly)ethers, in particular those commercialized by Solvay under the tradename Galden^{®} 55HT and hydrofluorocarbons, in particular 1,1,1,3,3-pentaftuorobutane.

In the process according to the invention, the concentration of the organic compound is generally from 0.01 to 5, preferably from 0.1 to 0.6 mole/liter solvent, relative to the feed volume of solvent.

In the process according to the invention, the elemental fluorine is generally supplied to the reaction as fluorine/inert gas mixture containing from 0.1 to 10 % vol, preferably from 2 to 5 % vol of F2.

In the process according to the invention, the molar ratio of fluorine to organic compound is generally equal to or greater than 0.8, preferably equal to or greater than 0.9. In the process according to the invention, the molar ratio of fluorine to organic compound is generally equal to or lower than 2, preferably equal to or lower than 1.5. Most preferably this ratio is about 1.

In the process according to the invention, the reaction is generally carried out at a temperature equal to or higher than -80°C preferably equal to or higher than -20°C. In the process according to the invention, the reaction is generally carried out at a temperature equal to or lower than +30°C preferably equal to or lower than 0°C.

The process according to the invention is generally carried out continuously.

The process according to the invention is generally carried out in a microreactor which is stable in contact with the components of the reaction medium, in particular elemental fluorine. Suitable materials which can be used for the parts of the microreactor supposed to enter in contact with the reaction medium include fluoropolymers, in particular polytetrafluoroethylene Hastelloy^{®} Steel and monel metal.

In the process according to the invention, after the reaction with elemental fluorine, the reaction medium can suitably treated with an aqueous solution to remove optionally formed HF and optional excess fluorine.

In a particular aspect, the process according to the invention, further comprises eliminating HF from the fluoroorganic compound, in particular an α,β-difluoro-addition product as described above to obtain a fluoroolefin. Elimination reactions can be carried out for example according to the procedures disclosed in EP-A-383128 and US 7,304,191, the relevant contents of which are incorporated by reference into the present patent application. In particular, elimination can be carried out by adding a base, for example a N-heterocycle, such as pyridine. Elimination can also be carried out thermally. For example, a solution of fluoroorganic compound can be heated in controlled manner to eliminate HF. The elimination reactions are preferably carried out in the presence of a stabilizer. BHT (2,6-di-tert-butyl-4-methylphenol) or hydroquinones. If the elimination is carried out in the absence of a base, HF can be removed from the reaction medium of the elimination reaction, for example, by distillation or by stripping with an inert gas, such as in particular nitrogen.

In a preferred embodiment of this aspect, the elimination of HF is carried out without isolating the fluoroorganic compound, in particular an α,β-difluoro-addition product.

In still another preferred embodiment of this aspect, the elimination of HF is carried out in a microreactor.

The example hereafter is understood to illustrate the invention, without however limiting it.

### Example

1 mole acrylic acid methyl ester is dissolved in 100 mL Galden^{®} HT 55. The solution, is reacted with F2 (1 vol % in N2) reacted in a static mixer ("cascade-type mixer in Hastelloy steel, obtainable by Ehrfeld Mikrotechnik BTS GmbH, Wendelsheim, Germany), followed by a microreactor (sandwich type reactor in Hastelloy, obtainable by Ehrfeld Mikrotechnik BTS GmbH ; volume = 30 ml).

F2 is charged in a molar ratio of 1 :1, relative to the acylic acid ester applied, into the micromixer. The feeding rate of the F2 is controlled via a rotameter and the feeding rate of acrylic ester solution, is controlled via a dosing pump.

The mixing of the gaseous/liquid phase is achieved in the micromixer (a split and recombine micromixer), the temperature of which is controlled at 0°C. After passing the micromixer the reaction mixture is fed into the sandwich type microreactor, which can be controlled in temperature, in order to provide the desired residence time. At the exit of the sandwich type microreactor a sample is taken showing substantial yield of 2,3-difluoropropionic acid methyl ester.

## Claims

1. Process for the manufacture of a fluoroorganic compound, which comprises elemental fluorine with an organic compound comprising at least one non-(hetero)aromatic unsaturated bond, in a microreactor in the presence of a solvent.

2. Process according to claim 1 wherein the organic compound comprises at least one olefinic double bond.

3. Process according to claim 2, wherein the organic compound corresponds to formula
HR1C=CR2-(C=O)-R3 (I)
wherein R1 and R2 are independently selected from H, optionally fluorinated, alkyl, (hetero)aryl, aralkyl and halogen and R3 is selected from, optionally fluorinated, alkyl, (hetero)aryl, aralkyl, OR4, wherein R4 is H, optionally fluorinated, alkyl, (hetero)aryl or aralkyl and NR5R6, wherein R5 and R6 are independently selected from H, optionally fluorinated, alkyl, (hetero)aryl and aralkyl.

4. Process according to claim 3, wherein the organic compound corresponds to formula
H2C=CH-(C=O)-OR4 (II)
wherein R4 is selected from H and, optionally fluorinated, methyl, ethyl, n-propyl, isopropyl, n-butyl, isobutyl and tert.butyl, preferably methyl.

5. Process according to anyone of claims 1 to 4, wherein the solvent is selected from the group consisting of perfluorocarbons, perfluoro(poly)ethers and hydrofluorocarbons.

6. Process according to anyone of claims 1 to 5, wherein the concentration of the organic compound is from 0.01 to 5, preferably from 0.1 to 0.6 mole/liter solvent, relative to the feed volume of solvent.

7. Process according to anyone of claims 1 to 6, wherein fluorine is supplied to the reaction as fluorine/inert gas mixture containing from 0.1 to 10 % vol, preferably from 2 to 5 % vol of F2.

8. Process according to anyone of claims 1 to 7, wherein the molar ratio of fluorine to organic compound is from 0.8 to 2, preferably from 0.9 to 1.5.

9. Process according to anyone of claims 1 to 8, wherein the reaction is carried out at a temperature of from -80°C to +30°C, preferably from -20°C to 0°C.

10. Process according to anyone of claims 1 to 9, wherein the microreactor comprises at least one microstructure heat exchanger.

11. Process according to anyone of claims 1 to 10, wherein, after the reaction with elemental fluorine, the reaction medium is treated with an aqueous solution to remove optionally formed HF and optional excess fluorine.

12. Process according to anyone of claims 1 to 11, wherein the fluororganic compound obtained through the reaction with elemental fluorine is an α,β-difluoro-addition product to the unsaturated bond,

13. Process according to claim 12, further comprising eliminating HF from the fluoroorganic compound to obtain a fluoroolefin.

14. Process according to claim 13, wherein the elimination of HF is carried out without isolating the α,β-diftuoro-addition product.

15. Process according to claim 13 or 14, wherein the elimination of HF is carried out in a microreactor.
